# EUROPEAN PATENT SPECIFICATION

(11) **EP 1 547 502 B1**
(45) Date of publication and mention of the grant of the patent: **17.10.2007**
(21) Application number: 03425819.4
(22) Date of filing: 23.12.2003
(51) Int. Cl.: A47K 3/28

(54) **Multisensorial sanitary appliance**
Multisensorische Sanitäreinrichtung
Dispositif sanitaire multisensoriel

(43) Date of publication of application: 29.06.2005
(73) Proprietor: TEUCO GUZZINI S.p.A., 62010 Montelupone (IT)
(72) Inventor: Guzzini, Mauro, 62019 Recanati (IT)
(74) Representative: Jorio, Paolo

(56) References cited:
- EP-A- 0 986 985
- EP-A- 1 298 259
- PATENT ABSTRACTS OF JAPAN vol. 015, no. 249 (M-1128), 25 June 1991 (1991-06-25) -& JP 03 079953 A (MATSUSHITA ELECTRIC IND CO LTD), 4 April 1991 (1991-04-04)

## Description

The present invention relates to a multisensorial sanitary appliance.

The present invention may be used to advantage in a shower stall, to which the following description refers purely by way of example.

Shower stalls featuring a number of high-pressure water jets for an invigorating massage effect are becoming increasingly popular. And to make showering even more enjoyable and relaxing, shower stalls equipped with music and sound reproducing and perfume dispensing devices have been proposed (as, for example, in Patent Application EP-0986985-A1).

Another proposal made in Patent Application EP-1298259-A2 is a hydromassage bath or shower stall in which water supply is regulated as a function of, and synchronized with, a music signal from an ordinary source (radio or CD player), and, at the same time, the music signal is also reproduced by a number of loudspeakers in the room in which the hydromassage bath or shower stall in installed.

As described in Patent Application EP-1298259-A2, a control device regulates water flow to the hydromassage bath or shower stall as a function of the amplitude of a control signal obtained by processing the music signal. Processing comprises first "band-pass" filtering the music signal to only select a few signal bands, and then "low-pass" filtering the signal to avoid operating the hydraulic water supply devices with signals of over 1 Hz frequency, which, as is known, do not enhance the overall effect and at the same time result in severe mechanical stress of the hydraulic devices.

In Patent Application EP-1298259-A2, the intention of the inventor in regulating water supply as a function of a music signal was supposedly to achieve a high degree of physical and, above all, mental relaxation, while at the same time enhancing body massage and toning action; and to make hydromassage bathing and showering more amusing and relaxing by hearing and feeling the music at the same time, which gives a much more heightened overall sense of well-being.

Various tests, however, have shown that a hydromassage bath or shower stall in accordance with Patent Application EP-1298259-A2 is not particularly effective.Indeed, in a hydromassage bath or a shower stall according to EP-1298259-A2 a particular track relative to one audio signal is always associated to the same track relative to one regulating pattern by which to time-regulate supply of the fluid by the fluid supply device. In such a way the treatment becomes repetitive and monotonous. In fact, EP-1298259-A2 at § [0041] states: "This device, by synchronizing the water and air jets with the tempo of the music, allows a powerful action producing physical and most of all mental relaxation, at the same time improving the body massage and toning action".

It is an object of the present invention to provide a multisensorial sanitary appliance designed to create a much more heightened feeling of well-being in the user.

According to the present invention, there is provided a multisensorial sanitary appliance as claimed in Claim 1 and preferably in any one of the following Claims depending directly or indirectly on Claim 1.

A non-limiting embodiment of the present invention will be described by way of example with reference to the accompanying drawings, in which:
Figure 1 shows a schematic view in perspective of a shower stall in accordance with the teachings of the present invention;
Figure 2 shows, schematically, a group of operating units of the Figure 1 shower stall.

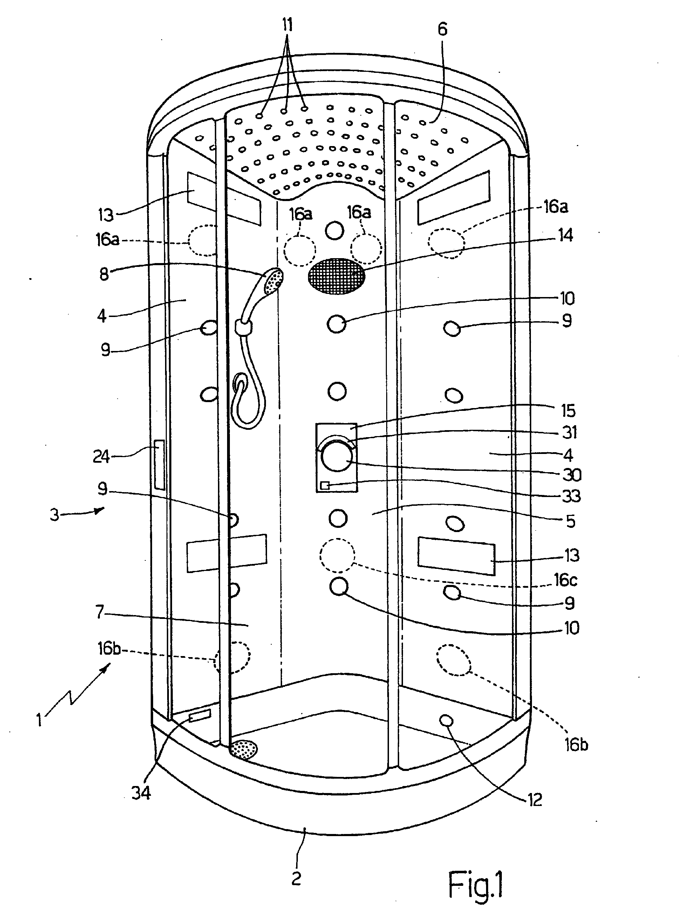

Number 1 in Figure 1 indicates as a whole a shower stall comprising a horizontal bottom wall defined by a shower stand 2, from which extend upwards four vertical lateral walls 3, 4, 5 bounded at the top by a substantially horizontal top wall 6. The two rear lateral walls 4 are connected on opposite sides to the front lateral wall 3, and are connected to each other by a further rear lateral wall 5. As shown, the front lateral wall 3 is transparent and made of plastic material or glass, while the rear lateral walls 4 and 5 are made of non-transparent plastic material.

A sliding door 7 is formed in front lateral wall 3, and is movable between a closed position and an open position to enable access to the inside of shower stall 1. Shower stall 1 is fitted inside with a hand-held shower 8; a number of nozzles 9 for emitting respective high-pressure water jets and fitted through rear lateral walls 4; a number of nozzles 10 for emitting respective high-pressure water jets and fitted through rear lateral wall 5; a number of holes 11 in top wall 6 for dispensing water in the form of drops; at least one opening 12 in shower stand 2 for producing a surf effect; a number of lamps 13 at both the top and bottom of shower stall 1; a perfume dispensing device 14; and a user interface panel 15.

In an embodiment not shown, shower stall 1 is provided with a central shower dispenser.

It is important to note that holes 11 are provided in large numbers, and are equally spaced in top wall 6 to create a "rainfall effect" inside shower stall 1; for which purpose, top wall 6 is simply the bottom of a container (not shown) filled with water. Hand-held shower 8 and nozzles 9, 10 obviously define water supply devices of shower stall 1.

Lamps 13 emit coloured light (even of different colours from one lamp to another) to define a special lighting effect fixture. In a different embodiment, at least some of lamps 13 are evenly spaced along the peripheral edge of top wall 6, and are oriented to direct the light downwards along lateral walls 3, 4 and 5.

In a further embodiment not shown, lamps 13 are integrated in or replaced by a single central lamp in a central portion of top wall 6.

Shower stall 1 also comprises a number of loudspeakers 16 for generating an audio signal inside shower stall 1. Each loudspeaker 16 is fitted to a respective wall 2-6, in the functional space inside shower stall 1, so as to be invisible, and so that its sound-emitting portion faces and is connected mechanically to wall 2-6.

It is important to note that loudspeakers 16 are located in different positions with respect to both a horizontal plane and a vertical plane, to obtain an actual three-dimensional sound. In other words, the user of shower stall 1 is able to distinguish the sound source in terms of "front", "back", "side", "top" and "bottom".

More specifically, in the Figure 1 embodiment, four loudspeakers 16a are all located in a first horizontal plane and evenly spaced along the periphery of shower stall 1, and two loudspeakers 16b are both located in a second horizontal plane, lower than the first horizontal plane, and also evenly spaced along the periphery of shower stall 1. A further sub-woofer type loudspeaker 16c is also provided for low sound reproduction. Location of the sub-woofer loudspeaker is not particularly important, in that low sounds have very little direction.

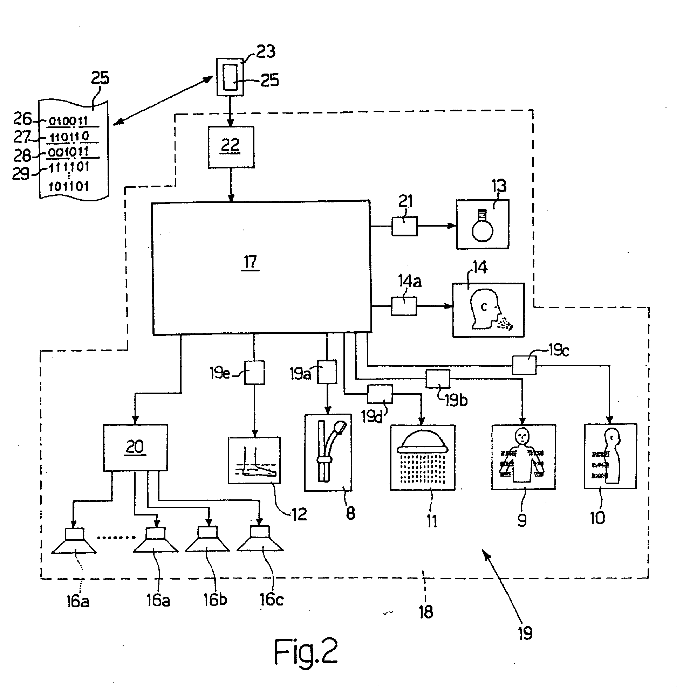

As shown in Figure 2, shower stall 1 comprises a control unit 17 housed in a concealed technical compartment 18 (shown schematically) integral with rear lateral walls 4 and 5. The technical compartment also houses a number of regulating members 19a-19e, each controlled by control unit 17 and for regulating water supply by a respective supply device 8-12.

Technical compartment 18 also houses a sound reproducing device 20 (Figure 2) controlled by control unit 17 and for driving loudspeakers 16a, 16b, 16c; and a regulating device 21 connected to control unit 17 and for driving and regulating illumination by lamps 13, by regulating both the intensity and colour of the light emitted.

Technical compartment 18 also houses a perfume dispensing device 14 controlled by a regulating device 14a.

As shown in Figure 2, control unit 17 is connected to a reading device 22, which receives a memory device 23 defined by a solid-state static memory, and in particular by a removable "Compact Flash" type memory board. Reading device 22 has a slot 24 (Figure 1) in which to insert memory device 23, and which is located outside shower stall 1, close to door 7.

In a different embodiment not shown, memory device 23 is defined by an optical disk.

Memory device 23 stores a file 25, which is structured in the form of a multitrack audio file and comprises at least one audio signal track 26, and at least one track 27 combined with track 26 and relative to a hydraulic pattern by which to time-regulate water supply by supply devices 8-12.

In actual use, control unit 17 reads file 25 to extract from it the combined tracks 26 and 27, and also drives regulating members 19a-19e to reproduce the hydraulic pattern defined by track 27, and drives sound reproducing device 20 to reproduce the audio signal defined by track 26.

In other words, track 26 contains information relative to an audio signal to be reproduced by sound reproducing device 20, and track 27 contains information relative to the hydraulic pattern by which to time-regulate water supply by supply devices 8-12. Control unit 17 thus reads tracks 26 and 27 simultaneously, so as to drive sound reproducing device 20 to reproduce the audio signal, and to drive regulating members 19a-19e to reproduce and combine the hydraulic regulating pattern with reproduction of the audio signal.

File 25 preferably also comprises a track 28 combined with track 26 and relative to an optical pattern by which to time-regulate illumination by lamps 13; and a track 28 combined with track 26 and relative to an olfactory pattern by which to time-regulate perfume emission.

In actual use, control unit 17 reads tracks 26-29 simultaneously to drive sound reproducing device 20 to reproduce the audio signal; to drive regulating members 19a-19e to reproduce and combine the hydraulic regulating pattern with reproduction of the audio signal; to drive regulating device 21 to reproduce and combine the optical pattern with reproduction of the audio signal; and to drive perfume regulating device 14a to reproduce and combine the olfactory pattern with reproduction of the audio signal.

In a preferred embodiment, track 27 relates to a number of hydraulic patterns by which to time-regulate water supply, and each associated with a respective supply device 8-12. In other words, control unit 17 drives each supply device 8-12 according to a respective hydraulic regulating pattern contained in track 27 and generally differing from those associated with the other supply devices 8-12.

Moreover, memory device 23 may contain a number of tracks 26, and a number of tracks 27-29, each combined with each track 26. Each track 26-29 differs slightly from the other tracks 26-29 of the same type, so as to define different variations of the same theme.

In actual use, control unit 17 reads one of tracks 26 and one of tracks 27-29, so that, by varying selection of tracks 26-29 each time from among those available, control unit 17 offers the user continual variations of the same theme, to prevent shower stall 1 from becoming repetitive and monotonous. The tracks 26-29 for use are selected automatically and substantially in random manner by control unit 17.

User interface panel 15 can also be used by the user of shower stall 1 to control combined regulating pattern reproduction. For example, user interface panel 15 can be used to stop, forward-feed or rewind combined regulating pattern reproduction at/to a given instant; to reproduce the same part of the regulating patterns continually; or to fade out regulating pattern reproduction gradually.

For example, user interface panel 15 comprises a knob 30, which can be turned to a number of angular positions to "travel" as required within a given "experience" mode. In other words, the user can go "forward" or "back" to re-experience any particularly pleasant sensations within the experience mode.

Knob 30 can also be connected mechanically to a button 33.

For example, one click of button 33 may correspond to a "start/pause" function; a double click of button 33 activates a repeat loop of a desired part of the experience; or a prolonged click (or three short clicks) of button 33 cuts off experience mode, and shower stall 1 operates in the normal way.

User interface panel 15 also comprises a display 31 curving about a sector of knob 30 and showing, for example, the various experience stages.

In the embodiment shown in the accompanying drawings, shower stand 2 is basin-shaped and can therefore be filled, in use, with a certain amount of water, the level of which is defined by an "overflow" type drain opening 34 located at a given height in the lateral wall of shower stand 2. Supply opening 12 is located lower down than drain opening 34 so as to be normally immersed, and is designed to produce waves in the water inside shower stand 2 and so produce the desired "surf effect".

In a further embodiment not shown, shower stand 2 comprises an adjustably rigid base wall; and a regulating device controlled by control unit 17 to regulate the rigidity of the base wall. In this case, control unit 17 reads from memory device 23 a further track synchronized with track 26 and relative to a mechanical pattern by which to time-regulate the rigidity of the base wall, and drives the regulating device according to the corresponding mechanical regulating pattern. For example, the base wall may be defined by a number of independent elastic containers filled with gel-like substances, and the regulating device may provide for regulating the pressure of the gel-like substances inside the containers.

In another embodiment not shown, the rigidity of the shower stand is regulated by exploiting the ability of various polymer materials to alter their viscosity alongside variations in the intensity of an electric (or magnetic) field to which they are subjected.

Obviously, an increase in the viscosity of the polymer material contained in the shower stand (and shielded by a layer of flexible material on which the user stands) corresponds to an increase in the rigidity of the shower stand.

An alternative embodiment (not shown) may comprise additional nozzles connected to a steam generator and controlled by control unit 17 to feed steam into the shower stall according to a corresponding time-regulating pattern stored in a further respective track in memory device 23.

As will be clear from the foregoing description, unlike known sanitary fixtures, the active components of shower stall 1 (supply devices 8-12, lamps 13, perfume dispensing device 14, loudspeakers 16a-16c) are all activated in combination with one another, as in a single orchestration, to immerse the user in a complete, complex, highly charged experience permitting a high degree of participation.

In other words, the user becomes part of a story, the script of which is written by the active components of shower stall 1. It should be pointed out that each active component of shower stall 1 is driven by control unit 17 to reproduce the relative regulating pattern, which is combined with but fully independent of the others, the regulating patterns being randomly combinable.

In other words, each regulating pattern, as opposed to being derived from another by mathematical methods, is created fully independently to combine synergically and randomly, with no repetition or monotony, with the others to achieve the maximum effect on the user.

It should be stressed that, using a number of tracks 26-29 stored in memory device 23 as described above, the user does not always experience exactly the same sensation, due to certain components changing preferably randomly each time, thus heightening the enjoyment of shower stall 1 by preventing it from becoming monotonous.

Moreover, files 25 are cheap, easy and fast to create and control, which can be done using any of numerous commercial softwares devised, for example, for MIDI technology.

In view of the considerable advantages afforded, the embodiments described above may obviously be applied to any type of sanitary appliance other than a shower stall, e.g. a hydromassage bath, or anywhere equipped for performing at least one sanitary treatment.

## Claims

1. A multisensorial sanitary appliance (1) comprising a number of supply devices (8-12) for supplying a fluid; at least one regulating member (19a-19e) for regulating supply of the fluid by the supply devices (8-12); and a sound reproducing device (20); wherein the sanitary appliance (1) comprises a control unit (17) for simultaneously reading at least one first track (26) relative to at least one audio signal, and at least one second track (27) relative to at least one regulating pattern by which to time-regulate supply of the fluid by the supply devices (8-12); the sanitary appliance (1) simultaneously driving the regulating member (19a-19e) to reproduce the regulating pattern regulating supply of the fluid, and the sound reproducing device (20) for reproducing the audio signal; the sanitary appliance (1) being **characterized by** the fact a memory device (23) contains a number of first tracks (26) and a number of second tracks (27); the control unit (17) randomly selecting and reading one of the first tracks (26) and one of the second tracks (27), and combining the two together.

2. A sanitary appliance as claimed in Claim 1, wherein the memory device (23) comprises a solid-state static memory.

3. A sanitary appliance as claimed in any one of the foregoing Claims, wherein the memory device (23) comprises a removable storage medium; the control unit (17) being connected to a reading device (22) compatible with the removable storage medium.

4. A sanitary appliance as claimed in Claim 3, wherein the removable storage medium is defined by an optical disk.

5. A sanitary appliance as claimed in Claim 3, wherein the removable storage medium is defined by a memory board.

6. A sanitary appliance as claimed in any one of the foregoing Claims, wherein the tracks in the memory device (23) are structured in the form of a multitrack audio file (25).

7. A sanitary appliance as claimed in any one of the foregoing Claims, and comprising a respective regulating member (19a-19e) for each supply device (8-12); the second track (27) comprising a corresponding regulating pattern for each supply device (8-12); and the control unit (17) driving each regulating member (19a-19e) according to the corresponding regulating pattern.

8. A sanitary appliance as claimed in any one of the foregoing Claims, and comprising a number of lighting devices (13) and a relative regulating member (21); the control unit (17) reading from the memory device (23) a third track (28) relative to at least one optical regulating pattern by which to time-regulate illumination by the lighting devices (13), and driving the lighting devices (13) by means of said regulating member (21) to reproduce the optical regulating pattern.

9. A sanitary appliance as claimed in any one of the foregoing Claims, and comprising at least one perfume dispensing device (14) and a relative regulating member (14a); the control unit (17) reading from the memory device (23) at least one fourth track (29) relative to at least one olfactory regulating pattern by which to time-regulate perfume emission, and driving the perfume dispensing device (14) by means of said regulating member (14a) to reproduce the olfactory regulating pattern.

10. A sanitary appliance as claimed in Claim 1, wherein the sanitary appliance is defined by a vertical shower stall (1); the sound reproducing device (20) comprising a number of loudspeakers (16a-16c) located in different positions with respect to a horizontal plane, and in different positions with respect to a vertical plane.

11. A sanitary appliance as claimed in Claim 10, wherein the sound reproducing device (20) comprises four first loudspeakers (16a), all located in a first horizontal plane and evenly spaced along the periphery of the shower stall (1); and two second loudspeakers (16b), both located in a second horizontal plane lower than said first horizontal plane; said two second loudspeakers (16b) being evenly spaced along the periphery of the shower stall (1).

12. A sanitary appliance as claimed in Claim 11, wherein the sound reproducing device (20) also comprises a sub-woofer type loudspeaker (16c) for low sound reproduction.

13. A sanitary appliance as claimed in Claim 1, wherein the sanitary appliance is defined by a vertical shower stall (1) having a substantially horizontal top wall (6) defining a ceiling of the shower stall (1); the top wall (6) comprising at least one respective supply device having a number of water supply holes (11) evenly spaced in the top wall (6).

14. A sanitary appliance as claimed in Claim 1, wherein the sanitary appliance is defined by a vertical shower stall (1) having a substantially horizontal bottom shower stand (2) defining a base of the shower stall (1); the shower stand (2) comprising at least one respective water supply device (12).

15. A sanitary appliance as claimed in Claim 14, wherein the shower stand (2) can be filled, in use, with a certain amount of water, the level of which is defined by overflow type drain means (34) located at a given height in a lateral wall of the shower stand (2); the supply device (12) of the shower stand (2) having an opening (12) for producing a "surf effect".

16. A sanitary appliance as claimed in any one of the foregoing Claims, wherein the sanitary appliance is defined by a vertical shower stall (1) having a substantially horizontal bottom shower stand (2) defining a base of the shower stall (1); the shower stand (2) comprising an adjustably rigid base wall whose rigidity is regulated by exploiting the ability of a polymer material to alter its viscosity alongside variations in the intensity of an electric (or magnetic) field to which it is subjected.

17. A sanitary appliance as claimed in Claim 16, wherein the control unit (17) reads from the memory device (23) a further track combined with the first track (26) and relative to at least one regulating pattern by which to time-regulate the rigidity of the base wall, and drives the regulating means of the viscosity of the polymer according to the corresponding regulating pattern.

18. A sanitary appliance as claimed in any one of the foregoing Claims, and also comprising a user interface panel (15) comprising a knob (30), which can be rotated to a number of angular positions to switch as required to a given multisensorial experience.

19. A sanitary appliance as claimed in Claim 18, wherein the knob (30) is connected to a button (33).

20. A sanitary appliance as claimed in Claim 19, wherein one click of said button (33) corresponds to a "start/pause" function, a double click of said button (33) activates a repeat loop of a desired part of the experience, and a prolonged click of said button (33) cuts off the multisensorial experience to activate substantially hydraulic operation of the shower stall (1).

## Patentansprüche

1. Multisensorische Sanitäreinrichtung (1) mit einer Anzahl an Zuführvorrichtungen (8-12) zum Zuführen eines Fluids, mindestens einem Regulierelement (19a-19e) zum Regulieren der Zuführung des Fluids durch die Zuführvorrichtungen (8-12) und einer Tonwiedergabevorrichtung (20), wobei die Sanitäreinrichtung (1) eine Steuereinheit (17) zum gleichzeitigen Lesen mindestens einer ersten Spur (26) relativ zu mindestens einem Audiosignal und mindestens einer zweiten Spur (27) relativ zu mindestens einem Reguliermuster aufweist, durch welches die Zuführung des Fluids durch die Zuführvorrichtungen (8-12) zeitlich reguliert wird, die Sanitäreinrichtung (1) das Regulierelement (19a-19e) zum Wiedergeben des Reguliermusters, welches die Zuführung des Fluids reguliert, und die Tonwiedergabevorrichtung (20) zum Wiedergeben des Audiosignals gleichzeitig antreibt und wobei die Sanitäreinrichtung (1) **gekennzeichnet ist durch** die Tatsache, dass eine Speichervorrichtung (23) eine Anzahl an ersten Spuren (26) und eine Anzahl an zweiten Spuren (27) enthält, wobei die Steuereinheit (17) eine der ersten Spuren (26) und eine der zweiten Spuren (27) zufällig auswählt und liest und die zwei miteinander kombiniert.

2. Sanitäreinrichtung nach Anspruch 1, wobei die Speichervorrichtung (23) einen statischen Festkörperspeicher aufweist.

3. Sanitäreinrichtung nach einem der vorangehenden Ansprüche, wobei die Speichervorrichtung (23) ein entfernbares Speichermedium aufweist, wobei die Steuereinheit (17) an einer Lesevorrichtung (22) angeschlossen ist, welche mit dem entfernbaren Speichermedium kompatibel ist.

4. Sanitäreinrichtung nach Anspruch 3, wobei das entfernbare Speichermedium durch eine Bildplatte definiert ist.

5. Sanitäreinrichtung nach Anspruch 3, wobei das entfernbare Speichermedium durch eine Speicherkarte definiert ist.

6. Sanitäreinrichtung nach einem der vorangehenden Ansprüche, wobei die Spuren in der Speichervorrichtung (23) in Form einer mehrspurigen Audiodatei (25) strukturiert sind.

7. Sanitäreinrichtung nach einem der vorangehenden Ansprüche, welche ein entsprechendes Regulierelement (19a-19e) für jede Zuführvorrichtung (8-12) aufweist, wobei die zweite Spur (27) ein entsprechendes Reguliermuster für jede Zuführvorrichtung (8-12) aufweist und die Steuereinheit (17) jedes Regulierelement (19a-19e) gemäß dem entsprechenden Reguliermuster antreibt.

8. Sanitäreinrichtung nach einem der vorangehenden Ansprüche, welche eine Anzahl an Beleuchtungsvorrichtungen (13) und ein relatives Regulierelement (21) aufweist, wobei die Steuereinheit (17) aus der Speichervorrichtung (23) eine dritte Spur (28) relativ zu mindestens einem optischen Reguliermuster liest, durch welches die Beleuchtung durch die Beleuchtungsvorrichtungen (13) zeitlich reguliert wird, und die Beleuchtungsvorrichtungen (13) mittels dem Regulierelement (21) antreibt, um das optische Reguliermuster wiederzugeben.

9. Sanitäreinrichtung nach einem der vorangehenden Ansprüche, welche mindestens eine Parfumabgabevorrichtung (14) und ein relatives Regulierelement (14a) aufweist, wobei die Steuereinheit (17) aus der Speichervorrichtung (23) mindestens eine vierte Spur (29) relativ zu mindestens einem olfaktorischen Reguliermuster liest, durch welches die Parfumabsonderung zeitlich reguliert wird, und die Parfumabgabevorrichtung (14) mittels dem Regulierelement (14a) antreibt, um das olfaktorische Reguliermuster wiederzugeben.

10. Sanitäreinrichtung nach Anspruch 1, wobei die Sanitäreinrichtung durch eine vertikale Duschkabine (1) definiert ist, die Tonwiedergabevorrichtung (20) eine Anzahl an Lautsprechern (16a-16c) aufweist, welche sich in Bezug auf eine horizontale Ebene an unterschiedlichen Positionen und in Bezug auf eine vertikale Ebene an unterschiedlichen Positionen befinden.

11. Sanitäreinrichtung nach Anspruch 10, wobei die Tonwiedergabevorrichtung (20) vier erste Lautsprecher (16a), welche sich alle in einer ersten horizontalen Ebene befinden und entlang dem Umfang der Duschkabine (1) gleichmäßig beabstandet sind, und zwei zweite Lautsprecher (16b) aufweist, welche sich beide in einer zweiten horizontalen Ebene befinden, welche niedriger als die erste horizontale Ebene ist, wobei die zwei zweiten Lautsprecher (16b) entlang dem Umfang der Duschkabine (1) gleichmäßig beabstandet sind.

12. Sanitäreinrichtung nach Anspruch 11, wobei die Tonwiedergabevorrichtung (20) auch einen Subwoofer-Lautsprecher (16c) zur Wiedergabe von tiefen Tönen aufweist.

13. Sanitäreinrichtung nach Anspruch 1, wobei die Sanitäreinrichtung durch eine vertikale Duschkabine (1) mit einer im Wesentlichen horizontalen oberen Wand (6) definiert ist, welche eine Decke der Duschkabine (1) definiert, wobei die obere Wand (6) mindestens eine entsprechende Zuführvorrichtung mit einer Anzahl an Wasserzuführöffnungen (11) aufweist, welche in der oberen Wand (6) gleichmäßig beabstandet sind.

14. Sanitäreinrichtung nach Anspruch 1, wobei die Sanitäreinrichtung durch eine vertikale Duschkabine (1) mit einem im Wesentlichen horizontalen, unteren Duschsockel (2) definiert ist, welcher einen Boden der Duschkabine (1) definiert, wobei der Duschsockel (2) mindestens eine entsprechende Wasserzuführvorrichtung (12) aufweist.

15. Sanitäreinrichtung nach Anspruch 14, wobei der Duschsockel (2) bei Verwendung mit einer bestimmten Menge an Wasser gefüllt werden kann, deren Pegel durch eine Überflussablaufeinrichtung (34) definiert ist, welche sich in einer vorgegebenen Höhe in einer Seitenwand des Duschsockels (2) befindet, wobei die Zuführvorrichtung (12) des Duschsockels (2) eine Öffnung (12) zum Erzeugen eines "Surfeffektes" aufweist.

16. Sanitäreinrichtung nach einem der vorangehenden Ansprüche, wobei die Sanitäreinrichtung durch eine vertikale Duschkabine (1) mit einem im Wesentlichen horizontalen, unteren Duschsockel (2) definiert ist, welcher einen Boden der Duschkabine (1) definiert, wobei der Duschsockel (2) eine verstellbare, steife Bodenwand aufweist, deren Steifigkeit durch das Ausnutzen der Fähigkeit eines Polymermaterials die Viskosität desselben entlang Variationen in der Stärke eines elektrischen (oder magnetischen) Feldes, welchem dasselbe ausgesetzt ist, zu ändern reguliert wird.

17. Sanitäreinrichtung nach Anspruch 16, wobei die Steuereinheit (17) aus der Speichervorrichtung (23) eine weitere Spur, welche mit der ersten Spur (26) kombiniert ist, und relativ zu mindestens einem Reguliermuster liest, durch welches die Steifigkeit der Bodenwand zeitlich reguliert wird, und die Einrichtung zum Regulieren der Viskosität des Polymers gemäß dem entsprechenden Reguliermuster antreibt.

18. Sanitäreinrichtung nach einem der vorangehenden Ansprüche, welche auch eine Benutzerschnittstellenkonsole (15) mit einem Drehknopf (30) aufweist, welcher in eine Anzahl an winkelstellungen gedreht werden kann, um nach Bedarf zu einem gegebenen multisensorischen Erlebnis zu schalten.

19. Sanitäreinrichtung nach Anspruch 18, wobei der Drehknopf (30) mit einem Knopf (33) verbunden ist.

20. Sanitäreinrichtung nach Anspruch 19, wobei ein Klick des Knopfes (33) einer "Start/Pause"-Funktion entspricht, ein Doppelklick des Knopfes (33) eine Wiederholungsschleife eines erwünschten Teils des Erlebnisses aktiviert und ein verlängerter Klick des Knopfes (33) das multisensorische Erlebnis abschaltet, um eine im Wesentlichen hydraulische Operation der Duschkabine (1) zu aktivieren.

## Revendications

1. Dispositif sanitaire multisensoriel (1) comprenant un certain nombre de dispositifs d'alimentation (8 à 12) destinés à alimenter en fluide ; au moins un élément de régulation (19a à 19e) destiné(s) à réguler l'alimentation en fluide par les dispositifs d'alimentation (8 à 12) ; et un dispositif de reproduction sonore (20) ; dans lequel le dispositif sanitaire (1) comprend une unité de commande (17) destinée à lire simultanément au moins une première piste (26) relative à au moins un signal audio et au moins une deuxième piste (27) relative à l'au moins un motif de régulation par lequel l'alimentation en fluide est régulée dans le temps par les dispositifs d'alimentation (8 à 12); le dispositif sanitaire (1) pilotant simultanément l'élément de régulation (19a à 19e) afin de reproduire le motif de régulation régulant l'alimentation en fluide et le dispositif de reproduction sonore (20) destiné à reproduire Je signal audio ; le dispositif sanitaire (1) étant **caractérisé en ce qu'**un dispositif de mémoire (23) contient un certain nombre de premières pistes (26) et un certain nombre de deuxièmes pistes (27); l'unité de commande (17) sélectionnant de manière aléatoire et lisant l'une des premières pistes (26) et l'une des deuxièmes pistes (27) et combinant les deux ensemble.

2. Dispositif sanitaire selon la revendication 1, dans lequel le dispositif de mémoire (23) comprend une mémoire statique à semi-conducteurs.

3. Dispositif sanitaire selon l'une quelconque des revendications précédentes, dans lequel le dispositif de mémoire (23) comprend un support de stockage amovible ; l'unité de commande (17) étant connectée à un dispositif de lecture (22) compatible avec le support de stockage amovible.

4. Dispositif sanitaire selon la revendication 3, dans lequel le support de stockage amovible est défini par un disque optique.

5. Dispositif selon la revendication 3, dans lequel le support de stockage amovible est défini par une carte mémoire.

6. Dispositif sanitaire selon l'une quelconque des revendications précédentes, dans lequel les pistes dans le dispositif de mémoire (23) sont structurées sous la forme d'un fichier audio à pistes multiples (25).

7. Dispositif sanitaire selon l'une quelconque des revendications précédentes, et comprenant un élément de régulation (19a à 19e) respectif pour chaque dispositif d'alimentation (8 à 12) ; la deuxième piste (27) comprenant un motif de régulation correspondant pour chaque dispositif d'alimentation (8 à 12) ; et l'unité de commande (17) pilotant chaque élément de régulation (19a à 19e) selon le motif de régulation correspondant.

8. Dispositif sanitaire selon l'une quelconque des revendications, et comprenant un certain nombre de dispositifs d'éclairage (13) et un élément de régulation (21) relatif; l'unité de commande (17) lisant depuis le dispositif de mémoire (23) une troisième piste (28) relative à au moins un motif de régulation optique par lequel l'éclairage est régulé dans le temps par les dispositifs d'éclairage (13), et pilotant les dispositifs d'éclairage (13) au moyen dudit élément de régulation (21) afin de reproduire le motif de régulation optique correspondant.

9. Dispositif sanitaire selon l'une quelconque des revendications, et comprenant au moins un dispositif de distribution de parfums (14) et un élément de régulation (14a) relatif ; l'unité de commande (17) lisant depuis le dispositif de mémoire (23) au moins une quatrième piste (29) relative à au moins un motif de régulation olfactif, et pilotant le dispositif de distribution de parfums (14) au moyen dudit élément de régulation (14a) afin de reproduire le motif de régulation olfactif.

10. Dispositif sanitaire selon la revendication 1, dans lequel le dispositif sanitaire est défini par une cabine de douche (1) verticale ; le dispositif de reproduction sonore (20) comprenant un certain nombre d'enceintes acoustiques (16a à 16c) localisées dans différentes positions par rapport à un plan horizontal, et dans différentes positions par rapport à un plan vertical.

11. Dispositif sanitaire selon la revendication 10, dans lequel le dispositif de reproduction sonore (20) comprend quatre premières enceintes acoustiques (16a), toutes localisées dans un premier plan horizontal et étant régulièrement espacées le long de la périphérie de la cabine de douche (1); et deux deuxièmes enceintes acoustiques (16b), toutes les deux localisées dans un deuxième plan horizontal inférieur audit premier plan horizontal ; lesdites deux deuxièmes enceintes acoustiques (16b) étant régulièrement espacées le long de la périphérie de la cabine de douche (1).

12. Dispositif sanitaire selon la revendication 11, dans lequel le dispositif de reproduction sonore (20) comprend également une enceinte acoustique (16c) du type de caisson de basse, pour une reproduction des basses.

13. Dispositif sanitaire selon la revendication 1, dans lequel le dispositif sanitaire est défini par une cabine de douche (1) verticale ayant une paroi supérieure (6) sensiblement horizontale définissant un plafond de la cabine de douche (1) ; la paroi supérieure (6) comprenant au moins un dispositif d'alimentation respectif ayant un certain nombre de trous d'alimentation en eau (11) régulièrement espacés dans la paroi supérieure (6).

14. Dispositif sanitaire selon la revendication 1, dans lequel le dispositif sanitaire est défini par une cabine de douche (1) verticale ayant une plateforme de douche inférieure (2) sensiblement horizontale définissant une base de la cabine de douche (1) ; la plateforme de douche (2) comprenant au moins un dispositif d'alimentation en eau (12) respectif.

15. Dispositif sanitaire selon la revendication 14, dans lequel la plateforme de douche (2) peut être remplie, en usage, d'une certaine quantité d'eau, le niveau de laquelle est défini par un moyen de vidange (34) du type de débordement, localisé à une hauteur donnée dans une paroi latérale de la plateforme de douche (2) ; le dispositif d'alimentation (12) de la plateforme de douche (2) ayant une ouverture (12) destinée à produire un « effet de vagues ».

16. Dispositif sanitaire selon l'une quelconque des revendications, dans lequel le dispositif sanitaire est défini par une cabine de douche (1) verticale ayant une plateforme inférieure de douche (2) sensiblement horizontale définissant une base de la cabine de douche (1) ; la plateforme de douche (2) comprenant une paroi de base à rigidité réglable, la rigidité de laquelle est régulée en exploitant la capacité d'une certaine matière polymérique à modifier sa viscosité ensemble avec des variations dans l'intensité d'un champ électrique (ou magnétique) auquel elle est soumise.

17. Dispositif sanitaire selon la revendication 16, dans lequel l'unité de commande (17) lit depuis le dispositif de mémoire (23) une autre piste combinée avec la première piste (26) et relative à au moins un motif de régulation par lequel la rigidité de la paroi de base est régulée dans le temps, et pilote le moyen de régulation de la viscosité du polymère selon le motif de régulation correspondant.

18. Dispositif sanitaire selon l'une quelconque des revendications, et comprenant également un panneau d'interface utilisateur (15) comprenant un bouton tournant (30) qui peut subir une rotation vers un certain nombre de positions angulaires pour commuter, en tant que de besoin, à une expérience multisensorielle donnée.

19. Dispositif sanitaire selon la revendication 18, dans lequel le bouton tournant (30) est connecté à un bouton (33).

20. Dispositif sanitaire selon la revendication 19, dans lequel un clic dudit bouton (33) correspond à une fonction de « démarrage / pause », un double clic dudit bouton (33) active une boucle de répétition d'une partie souhaitée de l'expérience, et un clic prolongé dudit bouton (33) coupe l'expérience multisensorielle pour activer un fonctionnement sensiblement hydraulique de la cabine de douche (1).
